# EUROPÄISCHE PATENTSCHRIFT

(11) **EP 1 486 200 B1**
(45) Veröffentlichungstag und Bekanntmachung des Hinweises auf die Patenterteilung: **21.03.2012**
(21) Anmeldenummer: 04010734.4
(22) Anmeldetag: 06.05.2004
(51) Int. Cl.: A61Q 19/00, A61K 8/36, A61K 8/06, A61K 8/49, A61K 8/73, A61K 8/25

(54) **Kosmetische Zubereitungen mit stabilisierten Konservierungsmitteln**
Cosmetic compositions comprising stabilized preservatives
Compositions cosmétiques contenant des agents conservateurs stabilisés

(30) Priorität: 14.06.2003 DE 10326899
(43) Veröffentlichungstag der Anmeldung: 15.12.2004
(73) Patentinhaber: Beiersdorf AG, 20253 Hamburg (DE)
(72) Erfinder: Kröpke, Rainer, 22869 Schenefeld (DE); Heptner, Astrid, 22083 Hamburg (DE); von der Fecht, Stephanie, 22869 Schenefeld (DE); Nielsen, Jens, 24558 Henstedt Ulzburg (DE); Konz, Celina, 25474 Bönningstedt (DE)

(56) Entgegenhaltungen:
- EP-A- 0 749 749
- EP-A- 1 013 178
- DE-A- 19 922 538
- DE-A- 19 928 495
- US-A- 3 649 302
- US-A- 4 146 652
- US-A- 4 425 329
- US-A- 5 075 113
- US-A- 5 965 179
- US-A- 6 120 758

## Beschreibung

Die Erfindung betrifft kosmetische oder dermatologische Zubereitungen enthaltend Kaliumsorbat als Konservierungsmittel und auf Basis von W/O- oder W/S - Emulsionen un- bzw. mittelpolare Lipide als Stabilisierungsmittel.

Seit 1939 ist die antimikrobielle Wirkung der Sorbinsäure bekannt. Als Derivate der Sorbinsäure werden vor allem die Alkalisalze als Konservierungsmittel insbesondere im Nahrungsmittelbereich eingesetzt. Der besondere Vorteil der Alkalisorbate liegt in deren guten Wasserlöslichkeit. Sorbinsäure und ihr Kalium- bzw. Natriumsalz werden als Konservierungsmittel, zumeist in Anteilen von 0,05 - 0,2 Gew.%, für pharmazeutische und kosmetische Präparate empfohlen (E. u. P. Pelle, Gyogyszzereszet 15, 94 [1971] [hun]; C. A. 75, 52752 [1971]). Wie aus verschiedensten Untersuchungen bekannt ist, verhält sich die Sorbinsäure und die Alkalisorbate hinsichtlich der toxikologischen Eigenschaften im Gegensatz zu anderen Konservierungsmitteln äußerst günstig. Weder Sorbinsäure noch Kaliumsorbat sind cancerogen, mutagen oder teratogen.

Da Sorbinsäure und die Alkalisorbate als Konservierungmittel für Kosmetika verwendet werden, ist auch deren Hautverträglichkeit geprüft worden und keinerlei negative Eigenschaften gefunden worden. Die Eigenschaften der Sorbinsäure bzw. des Kaliumsorbat, das bevorzugt für die Konservierung sauereingestellter kosmetischer Mittel geeignet ist, findet sich beispielsweise in E. Lück u. K. Remmert, SÖFW 118, 699 [1992]. Die Weltgesundheitsorganisation WHO hat die Unbedenklichkeit der Sorbinsäure bzw. der Alkalisorbate dadurch dokumentiert, dass sie für die Stoffe den höchsten Wert der aktzeptablen täglich Aufnahmedosis von 25 mg/kg Körpergewicht für Konservierungsstoffe festgesetzt hat.

Aufgrund der physiologischen Unbedenklichkeit werden Sorbinsäure und die Alkalisorbate in konservierenden Erzeugnissen eingesetzt, die während der Produktion, Verarbeitung oder Gebrauch mit der menschlichen oder tierischen Haut in Berührung kommt. US6120758 beschreibt konservierungsmittelsysteme für topische Zusammensetzungen, die Benzylalkohol, Dinatrium-EDTA oder eine para-Hydroxybenzoe-säure mit Verstärkern wie z.B. Sorbinsäuresalzen und Phospholipid kombinieren.

Es ist bekannt, dass in Kosmetika sich Kaliumsorbat abbaut und einen sehr unangenehmen Geruch hinterläßt, der schon in Spuren wahrnehmbar ist. Diese oxidativen Abbauprodukte können auch zu Verfärbungsproblemen führen, so dass Kaliumsorbat alleine oder mit den üblichen Konservierungsmittel in Kosmetika nicht einsetzbar ist. Bekannt ist ferner, das zur Vermeidung dieser Unannehmlichkeiten kaliumsorbat-haltiger Kosmetika der Zubereitung Stabilisatoren wie Allantoin oder BHT zugesetzt werden. BHT ist ein künstlich hergestelltes Antioxidations- und Konservierungsmittel. BHT wird als Toluolderivat toxisch nicht ganz unkritisch gesehen und wird nur noch in Ausnahmefällen in Kosmetika eingesetzt. Insbesondere sollte BHT nicht zum Einsatz kommen, wenn Kosmetika für Allergiker konzipiert werden.

Allantoin ist ein hautglättender Wirkstoff mit nur geringem Stabilisierungspotential, so dass der Einsatz als Stabilisierungsmittel für Konservierungsmittel nicht geeignet ist.

Aufgabe der vorliegenden Erfindung ist es daher kosmetische oder dermatologische Zubereitungen bereit zu stellen, die Kaliumsorbat als Konservierungsmittel enthalten und dennoch die Nachteile der aus dem Stand der Technik bekannten Kaliumsorbat-haltigen Zubereitungen nicht aufweisen. Insbesondere ist es Aufgabe der vorliegenden Erfindung Zubereitungen bereit zu stellen, die dabei nur geringe Mengen an Konservierungsmittel und zugesetzten Stabilisatoren enthalten.

Weitere Aufgabe der vorliegenden Erfindung ist es auch eine Alternative zu den bekannten kosmetischen Zubereitung bereit zu stellen, die den Stand der Technik bereichert.

Gelöst werden die angeführten Aufgaben durch eine Zubereitung entsprechend den Hauptansprüchen. Des weiteren umfasst die Erfindung die Verwendung derartiger Zubereitungen.

Es hat sich überraschend gezeigt, und darin liegt die Lösung aller Aufgaben, dass kosmetische oder dermatologische Zubereitungen enthaltend Kaliumsorbat und , wenn die Zubereitung auf einer W/O- oder W/S - Emulsion basiert, Lipide mit einer Grenzflächenspannung von größer oder gleich 10 mN/m sowie einen Citrat- und/oder Lactatpuffer gemäß Anspruch 1 enthalten, hervorragende Konservierungseigenschaften aufweisen. Die erfindungsgemäßen Zubereitungen weisen insbesondere die zahlreichen Nachteile der Kaliumsorbat-haltigen Zubereitungen aus dem Stand der Technik nicht auf. Es entstehen keinerlei Geruchs-oder Verfärbungsprobleme bei der Anwendung der erfindungsgemäßen Zubereitungen als Kosmetika.

Die Kombination von Kaliumsorbat als Konservierungsmittel mit den o.g. Stabilisatoren führt darüber hinaus zu sehr hautverträglichen Konservierungsmittelsystemen, so dass hautunfreundliche Systeme, wie beispielsweise Parabene, Germal II (Formaldehydabspalter), Kathon CG, abgelöst werden können.

In W/O- bzw. W/S-Zubereitungen, die ausschließlich unpolare- bis mittelpolare Lipidkomponenten enthalten, wie z.B. Isoeikosan, C5-Cyclomethicone, Mineralöl, wird eine Stabilisierungseigenschaft festgestellt. In solchen Zubereitungen entfaltet Kaliumsorbat eine gute mikrobiologische Wirkung und eine Oxidation zu unangenehm riechenden Stoffen unterbleibt bzw. wird deutlich hinausgezögert.

Kaliumsorbat kann vorteilhaft in Mengen von 0,001 bis 1 Gew.%, bevorzugt 0,01 bis 0,6 Gew.%, insbesondere bevorzugt 0,05 bis 0,2 Gew.%, bezogen auf die Gesamtmasse der Zubereitung zugesetzt sein. Die Zusatzmenge an Kaliumsorbat in Kosmetika ist zudem in machen Ländern gesetzlich geregelt.

Zusätzlich zu den erfindungsgemäßen Stabilisatoren können dem Fachmann bekannte Stabilisatoren weitere stabilisierende Substanzen der Zubereitung hinzugefügt werden, sofern sie die erfindungsgemäßen Vorteile nicht schmälern.
Als besonders vorteilhaft hat sich dabei der Zusatz von Salzen, insbesondere das Natriumsalz der Dehydracetsäure, und/oder Benzylalkohol erwiesen.

Synergistische Wirkung zeigt die Stabilisierung des Kaliumsorbats in Kombination mit Benzylalkohol und/oder dem Natriumsalz der Dehydracetsäure.

Da die Dehydracetsäure, (DHA, 3-Acetyl-6-methyl-2[H]-pyran-2,4[3H]-dion, 3[1-Hydroxyethyliden]-6-methyl-2[H]-pyran- 2,4[3H]-dion) der Strukturformel 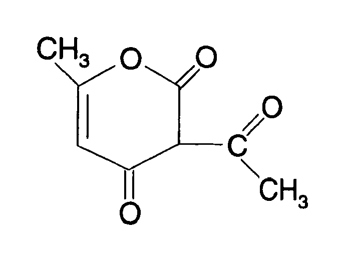 auch in höheren pH-Bereichen relativ gut wirksam ist, werden sie und ihre Salze zur Konservierung pharmazeutischer und kosmetischer Zubereitungen, insbesondere in Anteilen von 0,05-0,15 Gew.% empfohlen. Das Natriumsalz der Dehydracetsäure wird ebenfalls bevorzugt als Stabilisierungsmittelzusatz für die erfindungsgemäßen kosmetischen Zubereitungen verwendet. Hinweise auf dessen Eignung als Stabilisierungsmitel finden sich beispielsweise in C. A. Bennassi et al., Int. J. Cosmet. Sci. 10, 29 [1988]; C. A. 109, 155934 [1988].

Die bevorzugten Einsatzkonzentrationen an Salzen der Dehydracetsäure, bevorzugt das Natriumsalz betragen 0,05 - 1,0 Gew.%, an Benzylalkohol 0,1 - 2,0 Gew.%, bezogen auf die Gesamtmasse der Zubereitung.

Sehr gut synergistisch stabilisierend wirkt auch ein pH-Puffer im Bereich von 4-6. Dazu hat sich ein Citratpuffer bestehend aus Citronensäure : Natriumcitrat im Verhältnis 1:2 oder ein Lactat-Puffer bestehend aus Milchsäure : Natriumlactat im Verhältnis 1:2 erwiesen.

Diese Puffergemische werden den erfindungsgemäßen Zubereitung zugesetzt und erhöhen den Stabilisierungseffekt der erfindungsgemäß zugesetzten Stabilisatoren enorm. Die Wirksamkeit der Sorbate wird durch die Pufferung der Puffergemische deutlich erhöht, da die Sorbate die Zellwand durchdringen können und im Zellinneren ihre Wirkung entfalten können. Diese Eigenschaften werden durch ein saures Milieu erleichtert.

Als besonders vorteilhaft hat sich weiterhin herausgestellt, wenn die Zubereitung zusätzlich Elektrolyte in einer Menge von 0,1 - 5,0 Gew.%, bezogen auf die Gesamtmasse der Zubereitung, enthalten. Damit zeigen die erfindungsgemäßen Zubereitungen verbesserte anwendungsfreundliche Eigenschaften und insbesondere ist die Stabilisierung des Konservierungsmittels gesteigert. Als Elektrolyte sind Natriumchlorid und/oder Magnesiumsulfat bevorzugt.

Als erfindungsgemäß werden die Zubereitungen angesehen, die auf einer W/O-oder W/S - Emulsion basieren, die, neben Kaliumsorbat, Lipide mit einer Grenzflächenspannung von größer oder gleich 10 mN/m sowie einen Citrat- oder Lactatpuffer gemäß Anspruch 1 enthalten. Diese Lipide weisen erstaunlicherweise einen ebensolchen Stabilisierungseffekt für Kaliumsorbat in Kosmetika auf.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, dass die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.
Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Erfindung werden Lipide angesehen, deren Grenzflächenspannung gegen Wasser weniger als 10 mN/m beträgt, als unpolar solche, deren Grenzflächenspannung gegen Wasser mehr als 30 mN/m beträgt. Lipide mit einer Grenzflächenspannung gegen Wasser zwischen 10 und 30 mN/m werden im allgemeinen als mittelpolar bezeichnet.

Es gilt somit, dass Lipide mit einer Grenzflächenspannung von > 30 mN/m als unpolar, mit 10-30 mN/m als mittelpolar und mit < 10 mN/m als polar bezeichnet werden.

Polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen.

Die Ölphase der vorliegenden Emulsion kann daher gewählt werden aus der Gruppe der Dialkylether, der Gruppe der gesättigten oder ungesättigten, verzweigten oder unverzweigten Alkohole. Es ist insbesondere vorteilhaft, wenn die Ölphase einen Gehalt an C₁₂₋₁₅-Alkylbenzoat aufweist oder vollständig aus diesem besteht.

Ferner kann die Ölphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung 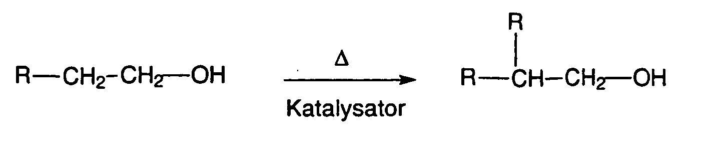 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur 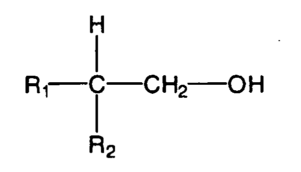 aus. Dabei bedeuten R₁ und R₂ in der Regel unverzweigte Alkylreste.

Erfindungsgemäß vorteilhaft werden der oder die Guerbet-Alkohole gewählt aus der Gruppe, bei denen
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl.

Erfindungsgemäß bevorzugte Guerbet-Alkohole sind das 2-Butyloctanol - es hat die chemische Struktur 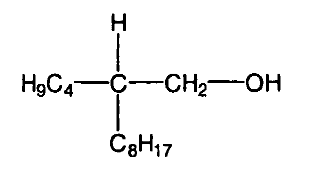 und ist beispielsweise unter der Handelsbezeichnung Isofol^{®} 12 von der Gesellschaft Condea Chemie GmbH erhältlich - und das 2-Hexyldecanol - es hat die chemische Struktur 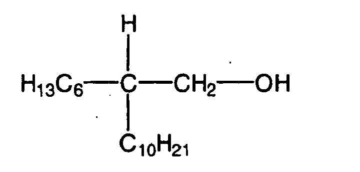 und ist beispielsweise unter der Handelsbezeichnung Isofol® 16 von der Gesellschaft Condea Chemie GmbH erhältlich. Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol® 14 erhältlich.

Die Gesamtmenge an Guerbet-Alkoholen in den fertigen kosmetischen oder dermatologischen Zubereitungen wird vorteilhaft aus dem Bereich bis 25,0 Gew.-%, bevorzugt 0,5 - 15,0 Gew.-% gewählt, bezogen auf das Gesamtgewicht der Zubereitungen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Besonders vorteilhafte mittelpolare Lipide im Sinne der vorliegenden Erfindung sind die im folgenden aufgelisteten Substanzen:

| **Handelsname** | **INCI-Name** | **Polarität** |
|---|---|---|
| | | [mN/m] |
| **DUB VCI 10** | Isodecyl Neopentanoate | 29,9 |
| **Dermol IHD** | Isohexyldecanoate | 29,7 |
| **Dermol 108** | Isodecyl Octanoate | 29,6 |
| **Dihexyl Ether** | Dihexyl Ether | 29,2 |
| **Dermol 109** | lsodecyl 3,5,5 Trimethyl Hexanoate | 29,1 |
| **Cetiol SN** | Cetearyl Isononanoate | 28,6 |
| **Isopropylpalmitat** | Isopropylpalmitat | 28,8 |
| **DC Fluid 345** | Cyclomethicone | 28,5 |
| **Dow Corning Fluid 244** | Cyclopolydimethylsiloxan | 28,5 |
| **Jojobaöl Gold** | | 26,2 |
| **Wacker AK 100** | Dimethicone | 26,9 |
| **Dermol 98** | 2- Ethylhexanosäure 3,5,5 Trimethylester | 26,2 |
| **Dow Corning Fluid 246** | Offen | 25,3 |
| **Eutanol G** | Octyldodecanol | 24,8 |
| **Isofol 16** | Hexyl Decanol | 24,3 |
| **Dermol 139** | Isotridecyl 3,5,5 Trimethylhexanonanoate | 24,5 |
| **Cetiol PGL** | Hexyldecanol (+) Hexyl Decyl Laurate | 24,3 |
| **Cegesoft C24** | Octyl Palmitate | 23,1 |
| **M.O.D.** | Octyldodeceyl Myristate | 22,1 |
| **Macadamia Nut Oil** | | 22,1 |
| **Silikonöl VP 112**0 | Phenyl Trimethicone | 22,7 |
| **Isocarb 12** | Butyt Octanoicacid | 22,1 |
| **Isopropylstearat** | Isopropyl Stearate | 21,9 |
| **Finsolv TN** | C12-15 Alkyl Benzoate | 21,8 |
| **Dermofeel BGC** | Butylene Glycol Caprylate/Caprate | 21,5 |
| **Miglyol 812** | Caprylic/Capric Triglyceride | 21,3 |
| **Trivent OCG** | Tricaprylin | 20,2 |
| **Dermol 866** | PEG " Diethylhexanoate/ Düsononanoate/ Ethylhexyl Isononanoate | 20,1 |
| **Isofol 14 T** | Butyl Decanol (+) Hexyl Octanol (+) Hexyl Decanol (+) Butyl Octanol | 19,8 |
| **Lipovol MOS-130** | Tridecyl Stearate(+) Tridecyl Trimellitate(+) 19,4 Dipentaerythrityl Hexacaprylate/Hexacaprate | 19,4 |
| **Ricinusoel** | | 19,2 |
| **Isofol Ester 0604** | | 19,1 |
| **Miglyol 840** | Propylene Glycol Dicaprylate/Dicaprate | 18,7 |
| **Isofol 12** | Butyl Octanol | 17,4 |
| **Tegosoft SH** | Stearyl Heptanoate | 17,8 |
| **Avocadooel** | | 14,5 |
| **Cetiol B** | Dibutyl Adipate | 14,3 |
| **Dermol 488** | PEG 2 Diethylenhexanoate | 10,1 |

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Besonders vorteilhafte unpolare Lipide im Sinne der vorliegenden Erfindung sind die im folgenden aufgelisteten Substanzen:

| **Handelsname** | **INCI-Name** | **Polarität** |
|---|---|---|
| | | [mN/m] |
| **Ecolane 130** | Cycloparaffin | 49,1 |
| **Nexbase 2006 FG** | Polydecene | 46.7 |
| **Polysynlane** | Hydrogenated Polyisobutene | 44.7 |
| **Wacker Silikonöl AK 50** | Polydimethylsiloxan | 46,5 |
| **Solvent ICH** | Isohexadecane | 43.8 |
| **Pionier 2076** | Mineral Oil | 43.7 |
| **Pionier 6301** | Mineral Oil | 43.7 |
| **Wacker Silikonöl AK 35** | Polydimethylsiloxan | 42,4 |
| **Isoeikosan** | Isoeikosan | 41.9 |
| **Wacker Silikonöl AK 20** | Polydimethylsiloxan | 40,9 |
| **Isofol 1212 Carbonat** | | 40,3 |
| **Softcutol O** | Ethoxydiglycoi Oleate | 40,5 |
| **Lipodermanol OL** | Decyl Olivate | 40,3 |
| **Cetiol S** | Dioctylcydohexane | 39,0 |
| **Pionier 2071** | Mineral Oil | 38.3 |
| **Hydrobrite 1000 PO** | Paraffinum Liquidum | 37,6 |
| **Tegosoft HP** | Isocetyl Palmitate | 36,2 |
| **Isofol Ester 1693** | | 33,5 |
| **Isofol Ester 1260** | | 33,0 |
| **Dow Corning Fluid 245** | Cyclopentasiloxan | 32,3 |
| **Prisorine 2036** | Octyl Isostearate | 31.6 |
| **Cetiol CC** | Dicaprylyl Carbonate | 31,7 |
| **Dermol 99** | Trimethylhexyl Isononanoate | 31,1 |
| **Dermol 89** | 2- Ethylhexyl Isononanoate | 31,0 |
| **Cetiol OE** | Dicaprylyl Ether | 30,9 |
| **Dihexylcarbonat** | Dihexyl Carbonate | 30,9 |
| **Silkflo 366 NF** | Polydecene | 30,1 |
| **Estol 1540 EHC** | Octyl Cocoate | 30.0 |

Es ist jedoch auch vorteilhaft, Gemische aus höher- und niederpolaren Lipiden, d.h. eine Mischung aus unpolaren und mittelpolaren Lipiden und dergleichen zu verwenden. So kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr, sofern die im Hauptanspruch geforderten Bedingungen eingehalten werden.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montan-wachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), und Syncrowax AW 1C (C₁₈₋₃₆ - Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkyl-hydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan sofern die im Hauptanspruch geforderten Bedingungen eingehalten werden.

Erfindungsgemäß können die Fett- und/oder Wachskomponenten sowohl einzeln als auch im Gemisch vorliegen. Auch beliebige Abmischungen solcher ÖI- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C₁₂₋₁₅-Alkylbenzoat, Capryl-Caprinsäure-triglycerid, Dicaprylylether sofern die im Hauptanspruch geforderten Bedingungen eingehalten werden.

Besonders vorteilhaft sind Mischungen aus Octyldodecanol, Capryl-Caprinsäure-triglycerid, Dicaprylylether, Dicaprylyl Carbonat, Cocoglyceriden, oder Mischungen aus C₁₂₋₁₅-Alkybenzoat und 2-Ethylhexylisostearat, Mischungen aus C₁₂₋₁₅-Alkybenzoat und Butylen Glycol Dicaprylat/Dicaprat sowie Mischungen aus C₁₂₋₁₅-Alkybenzoat, 2-Ethylhexylisostearat und Isotridecylisononanoat sofern die im Hauptanspruch geforderten Bedingungen eingehalten werden.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden. sofern die im Hauptanspruch geforderten Bedingungen eingehalten werden.

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

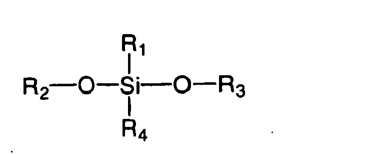

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind.

Als erfindungsgemäß vorteilhaft einzusetzenden linearen Silicone mit mehreren Siloxyleinheiten werden im allgemeinen durch Strukturelemente charakterisiert wie folgt: 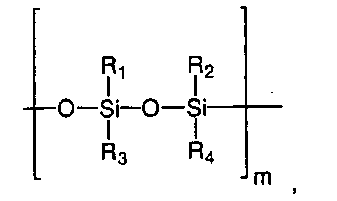 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten Verbindungen dieser Gruppe darstellen und sich durch die folgende Strukturformel auszeichnen 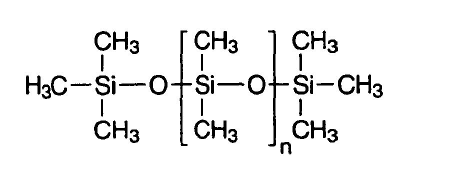 werden auch als Polydimethylsiloxan bzw. Dimethicon (INCI) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

Besonders vorteilhafte Polyorganosiloxane im Sinne der vorliegenden Erfindung sind ferner beispielsweise Dimethylpolysiloxane [Poly(dimethylsiloxan)], welche z. B. unter den Handelsbezeichnungen ABIL 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silicone (Octamethylcyclotetrasiloxan bzw. Decamethylcyclopentasiloxan), welche nach INCI auch als Cyclomethicone bezeichnet werden, aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind.

Besonders vorteilhaft im Sinne der vorliegenden Erfindung sind ferner die im folgenden aufgelisteten Silikonöle:

| Handelsname | INCI-Name | Polarität [mN/m] |
|---|---|---|
| Wacker Silikonöl AK 100 | Polydimethylsiloxan | 26,9 |
| Wacker Silikonöl AK 50 | Polydimethylsiloxan | 46,5 |
| Wacker Silikonöl AK 35 | Polydimethylsiloxan | 42,4 |
| Wacker Silikonöl AK 20 | Polydimethylsiloxan | 40,9 |
| Dow Corning Fluid 245 | Cyclopentasiloxan | 32,3 |
| Dow Corning Fluid 345 | Cyclomethicone | 28,5 |

Erfindungsgemäß vorteilhaft einzusetzende cyclische Silicone werden im allgemeinen durch Strukturelemente charakterisiert, wie folgt 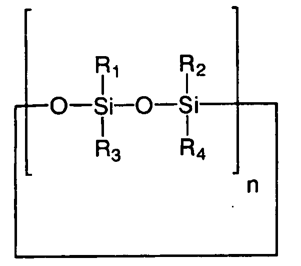 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, daß die Anzahl der unterschiedlichen Reste nicht notwendig auf bis zu 4 beschränkt ist). n kann dabei Werte von 3/2 bis 20 annehmen. Gebrochene Werte für n berücksichtigen, daß ungeradzahlige Anzahlen von Siloxylgruppen im Cyclus vorhanden sein können.

Besonders vorteilhafte cyclische Silikonöle im Sinne der vorliegenden Erfindung sind Cyclomethicone, insbesondere Cyclomethicone D5 und/oder Cyclomethicone D6.

Vorteilhafte Silkonöle bzw. Silikonwachse im Sinne der vorliegenden Erfindung sind cyclische und/oder lineare Silikonöle und Silikonwachse.

Es ist besonders vorteilhaft im Sinne der vorliegenden Erfindung das Verhältnis von Lipiden zu Silikonölen in etwa wie 1 : 1 (allgemein x : y) zu wählen.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphe-nylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxan-polyalkyl-polyether-copolymere wie das Cetyl-Dimethicon-Copolyol sowie das Cetyl-Dimethicon-Copolyol (und) Polyglyceryl-4-lsostearat (und) Hexyllaurat.

Eine gegebenenfalls gewünschte Ölkomponente der kosmetischen oder dermatologischen Zubereitung - beispielsweise in Form von Reinigungsemulsionen - im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Auch beliebige Abmischungen solcher ÖI- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Besonders vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat. Von den Kohlenwasserstoffen sind Paraffinöl, Squalan und Squalen vorteilhaft im Sinne der vorliegenden Erfindung zu verwenden.

Vorteilhaft kann die Ölkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die Ölkomponente wird ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 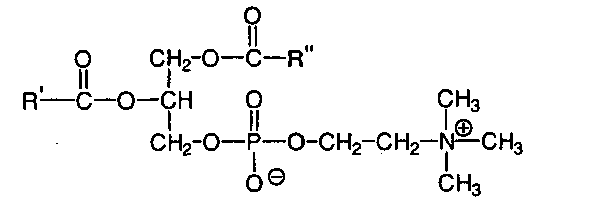 auszeichnen, wobei R' und R" typischerweise unverzweigte aliphatische Reste mit 15 oder 17 Kohlenstoffatomen und bis zu 4 cis-Doppelbindungen darstellen.

Die als vorteilhaft bezeichneten Lipide werden natürlich nur in den erfindungsgemäßen kosmetischen Zubereitungen verwendet sofern sie den Polaritätsanforderungen entsprechend den Ansprüchen genügen.

Der Vorteil der erfindungsgemäß verwendeten W/O- oder W/S Emulsionen führt dabei überaus anwendungsfreundlichen kosmetischen Zubereitungen.

W/O-Emulsionen zählen dabei zu den klassischen Emulsionen. Hierbei liegen die Wassertropfen als innere Phase vor und sind von Öl umgeben. W/O-Emulsionen ziehen nicht so schnell in die Haut ein. Sie hinterlassen einen schützenden Fettfilm, haben demzufolge eine okklusive Wirkung. Dieser Film reduziert den transepidermalen Wasserverlust (TEWL), sorgt so für eine intensive Hautbefeuchtung und stellt ein ausgeglichenes Fett-Feuchtigkeitsverhältnis her. Aufgrund ihrer genannten Merkmale sind die W/O-Emulsionen sehr effektiv bei trockenen Hautzuständen anzuwenden. Sie eignen sich gut zur Einschleusung von Wirkstoffen in die Haut und können nicht mit Wasser allein entfernt werden.

W/S-Emulsionen weisen mitunter einen sehr hohen Silikonölgehalt auf und zeigen meist ein sehr schönes, trockenes Hautgefühl.

Den erfindungsgemäßen Zubereitungen können Emulgatoren zugesetzt sein. Erfindungsgemäß können die Silikonemulgatoren vorteilhaft aus der Gruppe der Alkylmethiconcopolyole und/oder Alkyl-Dimethiconcopolyole gewählt werden, insbesondere aus der Gruppe der Verbindungen, welche gekennzeichnet sind durch die folgende chemische Struktur: 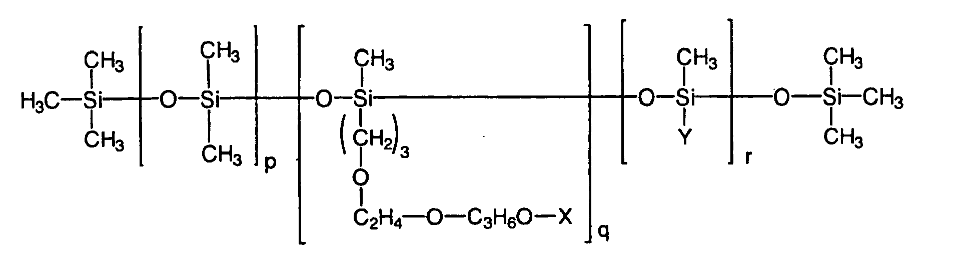 bei welcher X und Y unabhängig voneinander gewählt werden aus der Gruppe H (Wasserstoff) sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.

Ein Beispiel für besonders vorteilhaft im Sinne der vorliegenden Erfindung zu verwendende Silikonemulgatoren sind Dimethiconcopolyole, welche unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden.

Ein weiteres Beispiel für besonders vorteilhaft im Sinne der vorliegenden Erfindung zu verwendende grenzflächenaktiven Substanzen ist das Cetyl Dimethiconcopolyol, welches unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid erhältlich ist.

Ein weiterer vorteilhafter Silikonemulgator ist ,Octyl Dimethicon Ethoxy Glucosid' der Firma Wacker.

Der oder die W/O-Emulgatoren werden erfindungsgemäß vorzugsweise gewählt aus der folgenden Gruppe:

Sorbitanstearat, Sorbitanoleat, Lecithin, Glyceryllanolat, Lanolin, mikrokristallines Wachs (Cera microcristallina) im Gemisch mit Paraffinöl (Paraffinum liquidum), Ozokerit, hydriertem Ricinusöl, Glycerylisostearat, Polyglyceryl-3-Oleat, Wollwachssäuregemische, Wollwachsalkoholgemische, Pentaerythrithylisostearat, Polyglyceryl-3 Diisostearat, Sorbitan Oleat im Gemisch mit hydriertem Ricinusöl, Bienenwachs (Cera alba) und Stearinsäure, Natriumdihydroxycetylphosphat im Gemisch mit Isopropylhydroxycetylether, Methylglucosedioleat, Methylglucosedioleat im Gemisch mit Hydroxystearat und Bienenwachs, Mineralöl im Gemisch mit Petrolatum und Ozokerit und Glyceryloleat und Lanolinalkohol, Petrolatum im Gemisch mit Ozokerit und hydriertem Ricinusöl und Glycerylisostearat und Polyglyceryl-3-oleat, PEG-7-hydriertes Ricinusöl, Sorbitanoleat im Gemisch mit PEG-2-hydriertem Ricinusöl, Ozokerit und hydriertem Ricinusöl, Sorbitanisostearat im Gemisch mit PEG-2-hydriertem Ricinusöl, Polyglyceryl-4-isostearat, Polyglyceryl-4-isostearat, Hexyllaurat, Acrylat/ C₁₀₋₃₀-Alkylacrylat-Crosspolymer, Sorbitanisostearat, Poloxamer 101, Polyglyceryl-2-dipolyhydroxystearat, Polyglyceryl-3-Diisostearat, Polyglyceryl-4-dipolyhydroxystearat, PEG-30-dipolyhydroxystearat, Diiso-stearoylpolyglyceryl-3-diisostearat, Polyglyceryl-2-dipolyhydroxystearat, Polyglyceryl-3-dipolyhydroxystearat, Polyglyceryl-4-dipolyhydroxystearat, Polyglyceryl-3-dioleat.

Die erfindungsgemäßen Zubereitungen können weiterhin kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Die Menge der Verdickungsmittel beträgt beispielsweise 0,05 bis 5,0 Gew.-%, vorzugsweise 0,1 bis 3,0 Gew.-%, insbesondere 0,15 bis 2,0 Gew.-%, jeweils bezogen auf das Gesamtgewicht der Zubereitung.

Der Wassergehalt der Zubereitungen beträgt beispielsweise 60 bis 95 Gew.-%, vorzugsweise 75 bis 95 Gew.-%, insbesondere 80 bis 90 Gew.-%, jeweils bezogen auf das Gesamtgewicht der Zubereitung.

Aufgrund der hautfreundlichen Eigenschaften lassen sich die erfindungsgemäßen Zubereitungen für Kosmetika aller Art, insbesondere als Lotion, Creme aber auch als Tränkungslösung, Spray oder Aerosol verwenden.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

### W/O-Emulsionen (dünnflüssige W/O-Emulsionen auch zur Verwendung als Tränkunaslösung, Spray oder Aerosol)

| Beispiel | 1 | 2 | 3 | 4 | 5 |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,4 | 0,2 | 0,4 | 0,6 | 2,0 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,4 | 0,15 | 0,05 | 0,3 | 0,4 |
| Benzylalkohol | 0,3 | 0,4 | 0,25 | 0,15 | --- |
| Dehydracetsäure, Na-Salz | --- | --- | 0,05 | --- | 0,1 |
| Wasser | ad 100 | ad 100 | ad 100 | ad 100 | ad 100 |

### W/O-Emulsionen (dünnflüssige W/O-Emulsionen auch zur Verwendung als Tränkungslösung, Spray oder Aerosol)

| Beispiel | 6 | 7 | 8 | 9 | 10* |
|---|---|---|---|---|---|
| PEG-30 Dipolyhydroxystearat | --- | 0,5 | 0,25 | --- | 3,0 |
| Lanolin Alcohol | 1,0 | 1,5 | 1,75 | 3,0 | -- |
| Paraffinöl | 12,5 | 10,0 | 8,0 | 5,0 | 17,5 |
| Vaseline | 8,0 | 6,0 | 5,0 | 12,0 | 2,5 |
| hydrierte Kokosglyceride | 2,0 | 1,0 | 2,5 | 5,0 | 0,25 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulase | 0,5 | 1,0 | 0,75 | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Talkum, micronisiert | 1,0 | 0,5 | 5,0 | 0,25 | --- |
| Kaliumsorbat | 0,3 | 0,4 | 0,25 | 0,15 | --- |
| Iminodisuccinat | --- | --- | 0,05 | --- | 0,1 |
| Wasser | ad 100 | ad 100 | ad 100 | ad 100 | ad 100 |

| | | | | | |
|---|---|---|---|---|---|
| *nicht erfindungsgemäß | | | | | |

### W/S-Emulsionen (dünnflüssige W/S-Emulsionen auch zur Verwendung als Tränkunaslösung, Spray oder Aerosol)

| Beispiel | 11 | 12* | 13 | 14 | 15 |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 15,0 | -- | -- |
| Cyclomethicon | 12,5 | 15 | 18,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Milchsäure | 0,2 | --- | 1,75 | 0,3 | 1,0 |
| Natriumlactat | 0,4 | --- | 3,5 | 0,6 | 2,0 |
| Parfum | q,s, | q,s, | q,s, | q,s, | q,s, |
| Kaliumsorbat | 0,4 | 0,1 | 0,05 | 0,3 | 0,5 |
| Microcrystalline Cellulose | 1,0 | 0,1 | 0,5 | --- | --- |
| C5-Cyclomethicon | 1,0 | --- | 7,0 | --- | --- |
| lodopropynylbutylcarbamat | --- | --- | 0,05 | --- | 0,1 |
| modifizierte Stärke | --- | 2,5 | --- | 0,15 | -- |
| Wasser | ad 100 | ad 100 | ad 100 | ad 100 | ad 100 |

| | | | | | |
|---|---|---|---|---|---|
| *nicht erfindungsgemäß | | | | | |

### W/S-Emulsionen (dünnflüssige W/S-Emulsionen auch zur Verwendung als Tränkunaslösung, Spray oder Aerosol)

| Beispiel | 16 | 17 | 18 | 19 | 20 |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclomethicon | 12,5 | 15 | 28,0 | 25,0 | 17,5 |
| Dimethicon | 5,0 | 13,0 | 5,0 | 12,0 | 15,0 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| Kaliumsorbat | 0,4 | 0,1 | 0,05 | 0,3 | 0,4 |
| Microcrystalline Cellulose | 1,0 | --- | 1,5 | 2,5 | 0,1 |
| Benzylalkohol | 0,3 | 0,4 | 1,5 | 0,15 | --- |
| Stearyldimethicon | 0,5 | --- | 0,7 | --- | --- |
| Dehydracetsäure, Natrium-Salz | --- | --- | 0,05 | --- | 1,0 |
| modifizierte Stärke | --- | 2,5 | --- | 0,15 -- | |
| Wasser | ad 100 | ad 100 | ad 100 | ad 100 | ad 100 |

### W/O-Emulsionen (dünnflüssige W/O-Emulsionen auch zur Verwendung als Tränkungslösung, Spray oder Aerosol)

| Beispiel | 21 | 22 | 23 | 24 | 25 |
|---|---|---|---|---|---|
| PEG-22 Dodecyl Glycol Copolymer | 5,0 | 1,5 | 0,25 | --- | 3,0 |
| PEG-45 Dodecyl Glycol Polymer | 1,0 | 1,5 | 1,75 | 3,0 | -- |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Nachtkerzenöl | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| hydriertes Rizinusöl | 0,5 | 0,75 | 1,0 | 2,5 | 5,0 |
| Microcrystalline Cellulose | 0,5 | 1,0 | 0,75 | --- | --- |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Benzylalkohol | --- | --- | --- | 1,0 | 0,75 |
| Kaliumsorbat | 0,4 | 0,15 | 0,05 | 0,3 | 0,4 |
| Talkum, micronisiert | 0,3 | 0,4 | 2,5 | --- | --- |
| Iminodisuccinat | --- | --- | 0,05 | --- | 0,1 |
| Wasser | ad 100 | ad 100 | ad 100 | ad 100 | ad 100 |

### W/O-Emulsionen (dünnflüssige W/O-Emulsionen auch zur Verwendung als Tränkungslösung, Spray oder Aerosol)

| Beispiel | 26 | 27 | 28 | 29 | 30 |
|---|---|---|---|---|---|
| Polyglyceryl-2-dipolyhydroxystearat | 3,0 | --- | 0,25 | --- | 3,0 |
| Polyglyceryl-3 diisostearate | 1,0 | 3,5 | 1,75 | 2,5 | -- |
| PEG-40 sorbitanisostearate | --- | 2,5 | 0,5 | 3,5 | 3,0 |
| Paraffinöl | 12,5 | 10,0 | 8,0 | 5,0 | 17,5 |
| Isopropylstearat | 8,0 | 6,0 | 5,0 | 12,0 | 2,5 |
| hydrierte Kokosglyceride | 2,0 | 1,0 | 2,5 | 5,0 | 0,25 |
| Isopropylpalmitat | 0,5 | 1,0 | 0,75 | 3,0 | 0,25 |
| Dicaprylylcarbonat | 0,1 | 0,05 | 0,15 | 0,5 | 1,0 |
| Kaliumsorbat | 0,05 | 0,1 | 0,5 | 0,75 | 0,25 |
| hydriertes Rizinusöl | 0,5 | 0,75 | 1,0 | 2,5 | 5,0 |
| Microcrystalline Cellulose | 0,5 | 1,0 | 0,75 | --- | 0,1 |
| Talkum, micronisiert | 0,5 | 1,0 | --- | 0,25 | 0,1 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,09 | 0,2 | 0,3 | 1,0 |
| Natriumcitrat | 0,4 | 0,18 | 0,4 | 0,6 | 2,0 |
| Parfum | q,s, | q,s, | q,s, | q,s, | q,s, |
| 1,3 Butylenglykol | 2,0 | --- | 5,0 | --- | --- |
| Capryl-/Caprinsäuretriglycerid | 2,0 | 2,5 | 3,0 | 5,0 | 0,5 |
| Iminodisuccinat | --- | --- | 0,05 | --- | 0,1 |
| Wasser | ad 100 | ad 100 | ad 100 | ad 100 | ad 100 |

## Patentansprüche

1. Kosmetische oder dermatologische Zubereitung enthaltend Kaliumsorbat, **dadurch gekennzeichnet, dass** die Zubereitungen auf einer W/O- oder W/S - Emulsion basieren und als Stabilisierungsmittel Lipide mit einer Grenzflächenspannung von größer oder gleich 10 mN/m enthalten und ein Citratpuffer, bestehend aus Citronensäure Natriumcitrat im Verhältnis 1:2, und/oder ein Lactat-Puffer, bestehend aus Milchsäure : Natriumlactat im Verhältnis 1:2, enthalten ist.

2. Zubereitung nach Anspruch 1, **dadurch gekennzeichnet, dass** zusätzlich Dehydracetsaure und/oder deren Salze, insbesondere das Natriumsalz, in einer Menge von 0,01 bis 2 Gew.%, bevorzugt 0,05 bis 1 Gew.%, bezogen auf die Gesamtmasse der Zubereitung, enthalten ist.

3. Zubereitung nach einem der vorstehenden Ansprüche, **dadurch gekennzeichnet, dass** zusätzlich Benzylalkohol in einer Menge von 0,1 - 2,0 Gew.%, bezogen auf die Gesamtmasse der Zubereitung, enthalten ist.

4. Zubereitung nach einem der vorstehenden Ansprüche, **dadurch gekennzeichnet, dass** zusätzlich Elektrolyte, bevorzugt Natriumchlorid und/oder Magnesiumsulfat, in einer Menge von 0,1 - 5,0 Gew.%, bezogen auf die Gesamtmasse der Zubereitung, enthalten sind.

5. Verwendung der Zubereitung nach einem der Ansprüche 1 bis 4 als Kosmetikum, insbesondere als Lotion, Creme oder als Tränkungslösung, Spray oder Aerosol.

## Claims

1. Cosmetic or dermatological preparation comprising potassium sorbate, **characterized in that** the preparations are based on a W/O or W/S emulsion and comprise, as stabilizing agents, lipids with an interfacial tension greater than or equal to 10 mN/m and a citrate buffer consisting of citric acid:sodium citrate in the ratio 1:2, and/or a lactate buffer, consisting of lactic acid:sodium lactate in the ratio 1:2 is present.

2. Preparation according to Claim 1, **characterized in that** additionally dehydracetic acid and/or salts thereof, in particular the sodium salt, is present in an amount of from 0.01 to 2% by weight, preferably 0.05 to 1% by weight, based on the total mass of the preparation.

3. Preparation according to one of the preceding claims, **characterized in that** additionally benzyl alcohol is present in an amount of 0.1-2.0% by weight, based on the total mass of the preparation.

4. Preparation according to one of the preceding claims, **characterized in that** additionally electrolytes, preferably sodium chloride and/or magnesium sulphate, are present in an amount of 0.1-5.0% by weight, based on the total mass of the preparation.

5. Use of the preparation according to one of Claims 1 to 4 as cosmetic, in particular as lotion, cream or as impregnation solution, spray or aerosol.

## Revendications

1. Préparation cosmétique ou dermatologique contenant du sorbate de potassium, **caractérisée en ce que** les préparations sont à base d'une émulsion E/H ou E/S et contiennent en tant que stabilisants des lipides ayant une tension superficielle supérieure ou égale à 10 mN/m et un tampon citrate, constitué d'acide citrique : citrate de sodium en le rapport 1:2, et/ou un tampon lactate, constitué d'acide lactique : lactate de sodium en le rapport 1:2 est/sont contenu(s).

2. Préparation selon la revendication 1, **caractérisée en ce qu'**en outre de l'acide déhydracétique et/ou ses sels, en particulier le sel de sodium, est/sont contenu(s) en une quantité de 0,01 à 2 % en poids, de préférence de 0,05 à 1 % en poids, par rapport à la masse totale de la préparation.

3. Préparation selon l'une quelconque des revendications précédentes, **caractérisée en ce qu'**en outre de l'alcool benzylique est contenu en une quantité de 0,1 à 2,0 % en poids, par rapport à la masse totale de la préparation.

4. Préparation selon l'une quelconque des revendications précédentes, **caractérisée en ce qu'**en outre des électrolytes, de préférence du chlorure de sodium et/ou du sulfate de magnésium, sont contenus en une quantité de 0,1 à 5,0 % en poids, par rapport à la masse totale de la préparation.

5. Utilisation de la préparation selon l'une quelconque des revendications 1 à 4, en tant que cosmétique, en particulier en tant que lotion, crème ou en tant que solution d'imprégnation, composition pour vaporisation ou aérosol.
